# EUROPEAN PATENT APPLICATION

(11) **EP 2 433 556 A1**
(43) Date of publication of application: **28.03.2012**
(21) Application number: 11180173.4
(22) Date of filing: 06.09.2011
(51) Int. Cl.: A61B 5/00, A61B 1/06, A61N 5/06

(54) **Electronic endoscope system**

(30) Priority: 24.09.2010 JP 2010213998
(71) Applicant: Fujifilm Corporation, Minato-ku Tokyo (JP)
(72) Inventor: Yasuda, Hiroaki, Ashigarakami-gun, Kanagawa (JP); Endo, Azuchi, Ashigarakami-gun, Kanagawa (JP); Yamaguchi, Akira, Ashigarakami-gun, Kanagawa (JP); Saito, Maki, Ashigarakami-gun, Kanagawa (JP); Yamaguchi, Hiroshi, Ashigarakami-gun, Kanagawa (JP); Iida, Takayuki, Ashigarakami-gun, Kanagawa (JP)
(74) Representative: Klunker . Schmitt-Nilson . Hirsch

(57) **Abstract**

In photodynamic therapy (PDT), an LD (61) for normal light emission is continuously turned on, while an LD (62) for excitation light emission is turned off. An electronic shutter of a CCD (21) is closed in a first half (A) of a frame (FR), and is opened in a second half (B) to capture a PDT image. An LD (63) for therapeutic light emission is turned on in synchronization with a start of the frame (FR), and is turned off in the middle of the second half (B). Due to an overlap period (T3) established between a therapeutic light irradiation period (T1) and a charge accumulation period (T2), the PDT image has an irradiation spot of the therapeutic light overlaid on a normal image. Thus, the PDT is carried out in short possible treatment time with checking on a monitor (22) whether or not the therapeutic light is applied to an appropriate position.

## Description

### BACKGROUND OF THE INVENTION

### 1. Field of the Invention

The present invention relates to an electronic endoscope system for carrying out photodynamic diagnosis (PDD) and photodynamic therapy (PDT).

### 2. Description of the Related Art

Electronic endoscope systems are widely used in medical diagnosis and treatment. In recent years, there are known electronic endoscope systems that irradiate an internal body part not only with white light (normal light) for normal imaging, but also with special light in a specific narrow wavelength band to easily distinguish abnormal tissue including a tumor, a lesion, and the like. Photodynamic diagnosis (PDD) is known, by way of example, as one of diagnosis methods employing such an electronic endoscope system. What is more, there is known a system that performs photodynamic therapy (PDT) on the tumor found out by the PDD.

In the PDD, a photosensitive drug having high tumor affinity is administered in advance to a patient by intravenous injection or the like. The photosensitive drug has the property of gathering in tumor cells. For a lapse of predetermined time after the administration, the photosensitive drug is accumulated in the tumor cells in high concentration. After that, when excitation light having a predetermined wavelength is applied to an internal body part being suspected of having a tumor, the photosensitive drug accumulated in the tumor cells emits fluorescent light. Thus, the emission of the fluorescent light helps a doctor grasp the position and size of the tumor with high precision. The photosensitive drug having high tumor affinity includes hematoporphyrin derivative, which emits red fluorescent light having a wavelength of 635 nm, for example, upon application of blue excitation light near 405 nm.

The photosensitive drug used in the PDD produces active oxygen within the tumor cells by application of therapeutic light (for example, red light having wavelengths of 630 to 680 nm) , which has a wavelength different from that of the excitation light. The PDT is a therapeutic method that takes advantage of a cell-killing property of the active oxygen. The tumor cells having the accumulated photosensitive drug are destroyed by application of the therapeutic light. A treatment using an electronic endoscope does not need much time in general, but the PDT requires relatively long treatment time, typically one hour or more, though it depends on the size of the tumor and the power of the therapeutic light.

The therapeutic light emitted in the PDT has much higher power than that of the normal light. Thus, when the internal body part is imaged under the normal light during application of the therapeutic light, a phenomenon so-called halation occurs in which an irradiation area and its surrounding are white-out and blurred. For this reason, it is difficult to observe the body part during performance of the PDT. It is also difficult to carry out the PDD and the PDT at the same time, because the fluorescent light emitted in the PDD is in a wavelength band close to that of the therapeutic light. In spite of these facts, it is still desirable to observe the internal body part during the PDT, for the purpose of checking the irradiation area and a therapeutic effect of the therapeutic light while performing the PDT.

Against this backdrop, there is known an electronic endoscope system that synchronizes emission timing of the therapeutic light, the normal light, and the excitation light with actuation timing of an imaging device, to carry out in turn the PDT, the normal imaging, and the PDD. For example, Japanese Patent Laid-Open Publication No. 2006-130183 discloses a technique in which both the PDT and the PDD are compatible within a single frame by performing the PDT in a first field and the PDD in a second field. In Japanese Patent Laid-Open Publication No. 2006-094907, the normal imaging, the PDD, and the PDT are carried out in turn on a frame-by-frame basis, and accumulated electric charges are reset in the middle of the frame of the PDT to prevent occurrence of the halation.

In the case of destroying the tumor cells by the PDT, a total amount of the necessary therapeutic light depends on the size of the tumor cells and the like. Note that, according to a property of a light source, the amount of the therapeutic light emitted per time is constant. Therefore, time required for the PDT is determined by the amount of the therapeutic light emitted per time and the total amount of the therapeutic light necessary until completion of the therapy.

However, as described in the Japanese Patent Laid-Open Publication Nos. 2006-130183 and 2006-094907, when the normal imaging, the PDD, and the PDT are performed in turn on a frame (or field) basis, the emission of the therapeutic light is intermittently stopped. Therefore, the PDT needs long time until its completion. For example, if the PDT and the normal imaging are alternately performed on a frame-by-frame basis, treatment time is twice as long as that (minimum treatment time) in the case of emitting the therapeutic light continuously without intermission. Besides, if the PDT, the normal imaging, and the PDD are performed in turn on a frame-by-frame basis, the treatment time is triple as long as the minimum treatment time. Since the PDT typically needs one hour or more, as described above, doubling or tripling the treatment time significantly increases a burden of the patient.

On the other hand, resetting the accumulated electric charges in the middle of the frame of the PDT, as described in the Japanese Patent Laid-Open Publication No. 2006-094907, is effective at reducing the halation. In this case, however, an image captured after the reset becomes too dark to see at an area except for a portion near an irradiation spot of the therapeutic light where the halation used to occur.

### SUMMARY OF THE INVENTION

An object of the present invention is to provide an electronic endoscope system that can clearly observe an internal body part during performance of PDT with minimizing treatment time.

To achieve the above and other objects of the present invention, an electronic endoscope system according to the present invention includes a normal light emitting means, a therapeutic light emitting means, an imaging means, and a control means. The normal light emitting means emits white light to an internal body part to be imaged. The therapeutic light emitting means emits therapeutic light to the body part to treat a lesion emerging in the body part. The imaging means, which forms an image of the body part, has a plurality of pixels. Each pixel makes photoelectric conversion of incident light and accumulates signal charges of an amount corresponding to an amount of the incident light. The control means controls the normal light emitting means, the therapeutic light emitting means, and the imaging means, such that a therapeutic light irradiation period in which the body part is irradiated with the therapeutic light and a first charge accumulation period in which the signal charges are accumulated under irradiation of the white light overlap each other.

It is preferable that the control means establishes the therapeutic light irradiation period and the first charge accumulation period in an alternate manner. An overlap period is set up between the therapeutic light irradiation period and the first charge accumulation period at timing of transition from the therapeutic light irradiation period to the first charge accumulation period, or at timing of transition from the first charge accumulation period to the therapeutic light irradiation period.

The duration of the overlap period may be variable, and determined by the control means.

The control means may include a brightness judging means for judging brightness of the image captured by the imaging means. The control means determines the duration of the overlap period based on a judgment result of the brightness judging means.

The control means preferably adjusts the duration of the therapeutic light irradiation period to vary the duration of the overlap period.

The electronic endoscope system may further includes a special light emitting means for applying special light in a narrow wavelength band to the body part. The control means controls the special light emitting means and the imaging means, so as to establish a second charge accumulation period in which the signal charges are accumulated under irradiation of the special light.

The special light is preferably excitation light for causing a photosensitive drug accumulated in the body part to emit fluorescent light. The imaging means captures an image of the fluorescent light emitted from the photosensitive drug in the body part during a second charge accumulation period.

The control means may control the special light emitting means, the therapeutic light emitting means, and the imaging means, such that the therapeutic light irradiation period and the second charge accumulation period do not overlap each other.

While the lesion is treated by application of the therapeutic light, both imaging under irradiation with the white light and imaging under emission of the fluorescent light are preferably carried out. The imaging under irradiation with the white light and the imaging under emission of the fluorescent light may be carried out in an alternate manner.

The control means may establish both a first frame and a second frame in a treatment process of the lesion. The first frame includes the therapeutic light irradiation period, the first charge accumulation period, and the overlap period set up therebetween. The second frame includes the therapeutic light irradiation period and the second charge accumulation period without having an overlap period therebetween. The first frame and the second frame may be carried out in an alternate manner.

According to the present invention, it is possible to favorably check the treated body part during performance of photodynamic therapy (PDT) with minimizing an extension of treatment time.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is an external view of an electronic endoscope system;
Fig. 2 is a block diagram of the electronic endoscope system;
Fig. 3A is a sectional view of a light projecting unit of normal light;
Fig. 3B is a sectional view of a light projecting unit of excitation or therapeutic light;
Fig. 4 is a graph showing a property of a double notch filter;
Fig. 5 is a timing chart of normal light emission, therapeutic light emission, electronic shutter operation, and signal charge accumulation states of R-, G-, and B-pixels according to a first embodiment;
Fig. 6 is a timing chart of normal light emission, therapeutic light emission, electronic shutter operation, and signal charge accumulation states of R-, G-, and B-pixels according to a comparative example 2;
Figs. 7A to 7C are timing charts showing states in which an overlap period is varied;
Fig. 8 is a timing chart of an electronic endoscope system according to a second embodiment; and
Fig. 9 is a table showing the types of photosensitive drugs, and the wavelengths of excitation light, fluorescent light, and therapeutic light corresponding to each photosensitive drug.

### DETAILED DESCRIPTION OF THE PREFERRED EMBODIMENTS

### (First Embodiment)

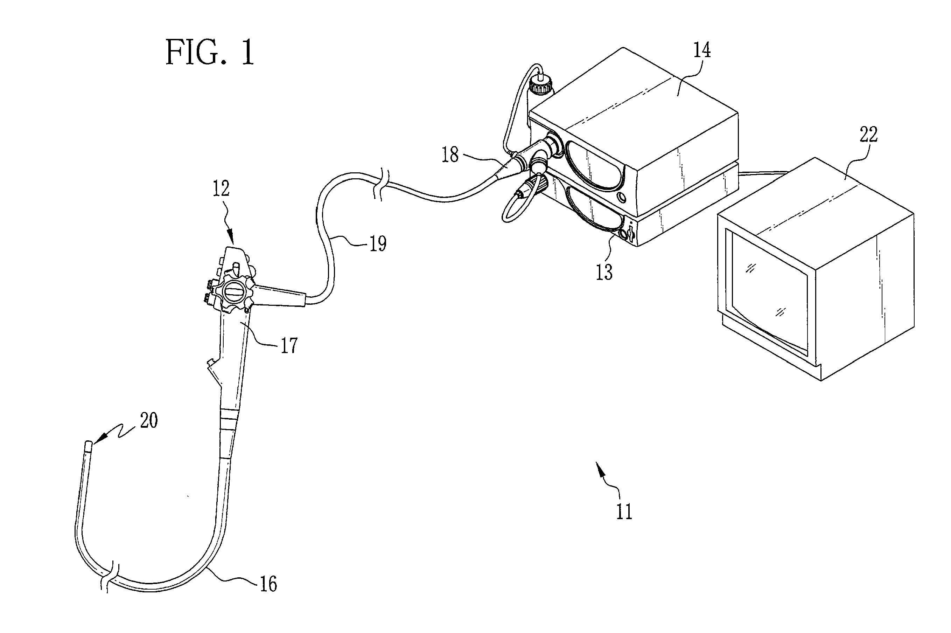

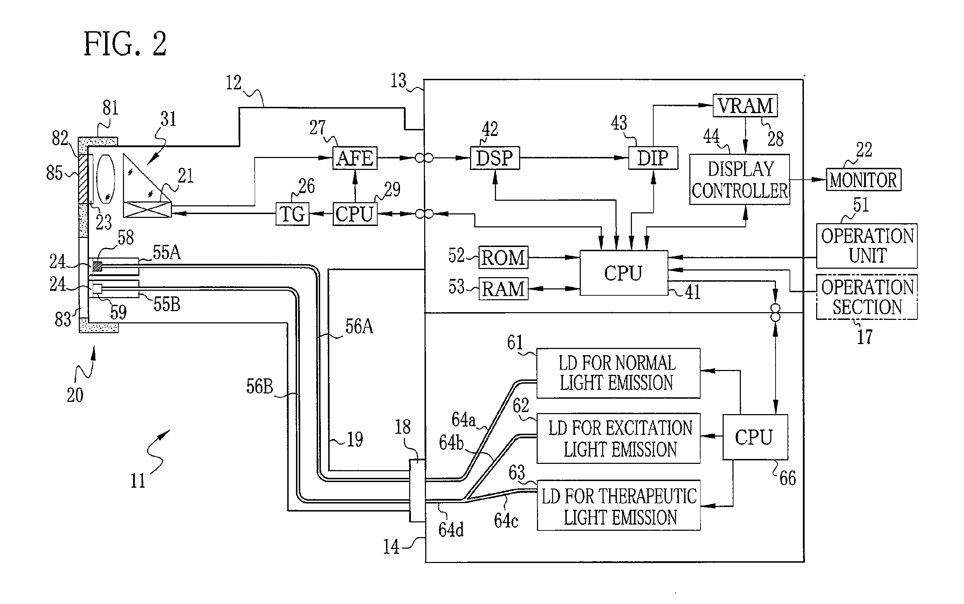

As shown in Fig. 1, an electronic endoscope system 11 is constituted of an electronic endoscope 12, a processor device 13, and a light source device 14. The electronic endoscope 12 includes a flexible insert section 16 to be introduced into a patient' s body cavity, an operation section 17 coupled to a base end of the insert section 16, a connector 18 connected to the processor device 13 and the light source device 14, and a universal cord 19 for connecting the operation section 17 to the connector 18. The insert section 16 contains a CCD image sensor (see Fig. 2; hereinafter simply called CCD) 21 at its distal end portion 20, to capture an image inside the body cavity.

On the operation section 17, there are provided an angle knob for flexibly bending the distal end portion 20 in a desired direction, an airing/watering button for ejecting air or water from a distal end surface of the insert section 16, a release button for capturing and storing a frame of an endoscopic image as a still image, a zoom button for zooming in or out on the endoscopic image displayed on a monitor 22, and a mode switching button for switching an operation mode between a normal imaging mode and a PDD mode. A medical instrument insertion port is provided on the operation section 17 at its distal end side. A medical instrument such as an electrocautery is inserted into the medical instrument insertion port, and led through a channel provided in the insert section 16, and out from a medical instrument outlet provided in the distal end surface.

The processor device 13 is electrically connected to the light source device 14. The processor device 13 performs centralized control of the electronic endoscope system 11. The processor device 13 supplies electric power to the electronic endoscope 12 through the universal cord 19 and a cable provided in the insert section 16, and controls operation of the CCD 21. Also, the processor device 13 receives an image signal outputted from the CCD 21 through the cable, and applies various types of image processing to the image signal to produce image data. The image data produced by the processor device 13 is displayed as the endoscopic image on the monitor 22 connected to the processor device 13.

As shown in Fig. 2, the distal end portion 20 contains the CCD 21, an objective optical system 31, light projecting units 55A and 55B, and the like. A timing generator (TG) 26, an analog frontend processor (AFE) 27, a VRAM 28, and a CPU 29 are provided inside the operation section 17, the connector 18 of the universal cord 19, and the like. The objective optical system 31 and the CCD 21 are disposed behind an imaging window 23, so that the objective optical system 31, including a lens group and a prism, forms an image of an internal body part on an imaging surface of the CCD 21. The internal body part is irradiated with illumination light and therapeutic light for photodynamic therapy (PDT) through lighting windows 24. The illumination light and the therapeutic light are supplied from the light source device 14 to the electronic endoscope 12 through optical fibers 56A and 56B, respectively, provided in the universal cord 19 and the insert section 16. The illumination light and the therapeutic light are applied from the light projecting units 55A and 55B disposed at a projection end through lighting lenses (not shown) to the body part.

The CCD 21 performs photoelectric conversion on the image of the internal body part, which is formed on the imaging surface of the CCD 21 by the objective optical system 31. The CCD 21 has a plurality of pixels, and each pixel outputs a pixel signal corresponding to an amount of incident light. The imaging surface has a light receiving area occupying the middle of the imaging surface, and an optical black area situated around the light receiving area. The light receiving area has an array of active pixels. In the light receiving area, a color filter having plural color elements is disposed on the array of the pixels. The color filter is, for example, an RGB primary color filter of the Bayer pattern, or a CMY or CMYG complementary color filter. In the optical black area, the pixels are shielded against light by a lightproof film, and output pixel signals corresponding to dark current noise. Thus, the image signal outputted from the CCD 21 includes the pixel signals outputted from the pixels in the light receiving area and the pixel signals outputted from the pixels in the optical black area. The pixel signals from the light receiving area are used to produce the endoscopic image, and the pixel signals from the optical black area are used in making a dark current correction to the endoscopic image. The CCD 21 operates in response to a clock signal inputted from the TG 26, to perform accumulation and readout of the signal charges. The duration of a period of accumulating the signal charges (hereinafter called charge accumulation period), the timing of starting or ending the charge accumulation period, and the like, in other words, opening and closing operation of an electronic shutter and its timing are regulated by input timing of the clock signal functioning as a shutter pulse. The electronic shutter is closed while the shutter pulse is inputted, and the electronic shutter is opened while the input of the shutter pulse is stopped.

The image signal outputted from the CCD 21 is an analog signal. The analog image signal is subjected to noise reduction processing and gain correction processing by the AFE 27, and subjected to A/D conversion. After that, the image signal is inputted to a digital signal processor (DSP) 42 of the processor device 13 through the universal cord 19 and the connector 18.

The TG 26 supplies the CCD 21 with the clock signal. The CCD 21 performs imaging operation in response to the clock signal inputted from the TG 26, and outputs the image signal. After the electronic endoscope 12 is connected to the processor device 13, the CPU 29 actuates the TG 26 based on an operation signal from a CPU 41 of the processor device 13. As described later on in detail, the clock signal outputted from the TG 26 is determined by the CPU 29. The clock signal commands to start or end the operation of the CCD 21, including the accumulation of the signal charges, at timing variously different in accordance with the operation mode such as a photodynamic therapy (PDT) mode or a photodynamic diagnosis (PDD) mode.

The AFE 27 includes a correlated double sampling circuit (CDS), an automatic gain control circuit (AGC), and an A/D converter. The CDS applies correlated double sampling processing to the image signal outputted from the CCD 21 to remove noise, which is caused by operation of the CCD 21. The AGC amplifies the image signal after the removal of the noise by the CDS. The A/D converter converts the image signal amplified by the AGC into digital image signal having a predetermined number of bits. The operation of the AFE 27 is controlled by the CPU 29. For example, the CPU 29 adjusts an amplification factor (gain) of the AGC based on the operation signal from the CPU 41 of the processor device 13.

The light projecting units 55A and 55B apply light led from the light source device 14 through the optical fibers 56A and 56B to the internal body part, respectively. Tip ends of the light projecting units 55A and 55B are sealed with protective glass plates (not shown) . The light projecting unit 55A is exposed from the distal end surface of the insert section 16 through the protective glass plate as the lighting window 24 to emit the normal light. The light projecting unit 55B is exposed from the distal end surface of the insert section 16 through the protective glass plate as the lighting window 24 to emit the excitation or therapeutic light.

Into the light projecting unit 55A for emitting the normal light, blue laser light is led from the light source device 14 through the optical fiber 56A. The light projecting unit 55A is provided with a phosphor 58 at its tip end. The phosphor 58, which is made from YAG or BAM (BaMgAl₁₀O₁₇) , for example, absorbs a part of the blue laser light projected from the optical fiber 56A, and emits green to yellow excitation light. Thus, the light projecting unit 55A projects pseudo-white light as the normal light formed by a combination of the blue light transmitted through and diffused across the phosphor 58 and the green to yellow excitation light emitted from the phosphor 58. An irradiation area of the normal light is substantially equal to or larger than an imaging area of the electronic endoscope 12. Therefore, the normal light almost evenly illuminates the entire surface of the endoscopic image.

Into the light projecting unit 55B to emit the excitation light for the PDD or the therapeutic light for the PDT, one of the excitation light and the therapeutic light is led through the optical fiber 56B. The light projecting unit 55B is provided with a light diffusing member 59 at its tip end to diffuse the light from the optical fiber 56B to a predetermined range. Thus, the light projecting unit 55B projects the light to an irradiation area of a certain expansion. The irradiation area of the excitation or therapeutic light is smaller than that of the normal light, so the excitation or therapeutic light is mainly applied to just a part of the imaging area of the electronic endoscope 12.

The processor device 13 has the CPU 41, the digital signal processor (DSP) 42, a digital image processor (DIP) 43, a display controller 44, an operation unit 51, and the like.

The CPU 41 is connected to each part through not-shown data buses, address buses, and control wires, to perform centralized control of the processor device 13. A ROM 52 stores various types of programs (OS, application programs, and the like) and various types of data (graphic data, and the like) to control operation of the processor device 13. The CPU 41 reads the necessary programs and data from the ROM 52, and loads the read programs and data to a RAM 53 being a working memory to execute them in succession. Also, the CPU 41 obtains information differing from examination to examination, which includes an examination date and data related to a patient and a doctor, from the operation unit 51 or through a network such as a LAN, and writes the information to the RAM 53.

Furthermore, the CPU 41 inputs a regulation signal to the CPU 29 of the electronic endoscope 12, to regulate the duration and start/end timings of the charge accumulation period of the CCD 21 in accordance with the operation mode, which indicates the type of light to be applied to the internal body part, light application timing, and the like. The CPU 29 of the electronic endoscope 12 drives the TG 26 in response to the regulation signal inputted from the CPU 41, so as to regulate the open/close timings of the electronic shutter of the CCD 21 and the like in accordance with the operation mode. In the case of imaging inside the body under the normal light while the insert section 16 is being inserted into the body cavity, for example, almost the entire duration of a single frame, which corresponds to a cycle of capturing the single endoscopic image, is assigned to the charge accumulation period, except for the duration of reading out the signal charges and outputting the image signal. On the other hand, in the case of imaging the internal body part during the performance of the PDT, the CPU 29 commands the electronic shutter to close in a first half of the single frame, and to open in a second half of the frame as the charge accumulation period.

The CPU 41 inputs the same regulation signal to a CPU 66 of the light source device 14, in addition to the CPU 29 of the electronic endoscope 12. Thus, the normal light, the excitation light, and the therapeutic light are emitted from the light source device 14 at appropriate timing in accordance with the operation mode. To synchronize the operation of the CCD 21 and LDs 61 to 63 contained in the light source device 14, the CPU 41 inputs a synchronization signal to the CPU 29 and the CPU 66 in accordance with the operation mode.

The DSP 42 applies various types of signal processing including color separation, color interpolation, gain correction, white balance adjustment, gamma correction, and the like to the image signal inputted from the CCD 21, to produce image data. The image data produced by the DSP 42 is inputted to a work memory of the DIP 43. The DSP 42 also produces ALC data, which is necessary for automatic light control (ALC) of an illumination light amount, such as an average luminance value being an average of luminance of every pixel in the produced image data, and inputs the ALC data to the CPU 41.

The DIP 43 applies various types of image processing such as electronic magnification, color enhancement processing, and edge enhancement processing to the image data produced by the DSP 42. The image data after being processed by the DIP 43 is temporarily written to the VRAM 28 as the endoscopic image, and is inputted to the display controller 44.

The display controller 44 selectively obtains the endoscopic image from the VRAM 28, and displays the image on the monitor 22. Also, the display controller 44 receives the graphic data stored on the ROM 52 and the RAM 53 through the CPU 41. The graphic data includes a display mask for covering an area other than an effective pixel area showing an object out of the endoscopic image, text data such as the examination date and the data related to the patient and doctor, and GUI. The display controller 44 applies image overlay processing, by which the graphic data is overlaid on the endoscopic image obtained from the VRAM 28. Then, the display controller 44 converts the processed image into a video signal (component signal, composite signal, and the like) that is compatible with a display format of the monitor 22, and outputs the video signal to the monitor 22. Thus, the endoscopic image is displayed on the monitor 22.

The operation unit 51 includes a pedal switch to start and stop emitting the therapeutic light of the PDT, in addition to well-known input devices such as a mouse, a keyboard, and an operation panel provided on a cabinet of the processor device 13. The CPU 41 actuates each part of the electronic endoscope system 11 in response to operation signals from the operation unit 51 and the operation section 17 of the electronic endoscope 12.

In addition to the above, the processor device 13 includes a compression circuit for compressing image data in a predetermined format (for example, JPEG format), a medium I/F for writing the compressed image data into a removable medium in response to a press of the release button, a network I/F for controlling transmission of various types of data through the network such as the LAN, and the like. These circuits are connected to the CPU 41 through the data buses and the like.

The light source device 14 has three types of laser diodes as light sources, that is, the LD 61 for normal light emission, the LD 62 for excitation light emission, and the LD 63 for therapeutic light emission. The CPU 66 controls operation of each LD 61, 62, or 63.

The LD 61 for normal light emission emits blue laser light having a center wavelength of 445 nm. The blue laser light is led into an optical fiber 64a through a not-shown lens and the like. The optical fiber 64a is connected to the optical fiber 56A of the electronic endoscope 12 via the connector 18. Thus, the blue laser light emitted from the LD 61 is led into the light projecting unit 55A, and is incident upon the phosphor 58. Upon entry of the blue laser light, the phosphor 58 emits the green to yellow excitation light. Thus, the combination of the blue laser light and the green to yellow excitation light is applied to the internal body part as the pseudo-white normal light.

The LD 62 for excitation light emission emits violet laser light having a center wavelength of 405 nm. The violet laser light is led into an optical fiber 64b through a not-shown lens and the like. The optical fiber 64b and an optical fiber 64c are integrated into a single optical fiber 64d by a coupler (not shown) , so the optical fiber 64b is connected to the optical fiber 56B of the electronic endoscope 12 via the connector 18. Thus, the violet laser light emitted from the LD 62 is led into the light projecting unit 55B. The violet laser light is diffused by the light diffusing member 59, and is applied to the internal body part as the excitation light for the PDD. Upon application of the excitation light, a photosensitive drug used in the PDD and PDT emits red fluorescent light having a center wavelength of approximately 660 nm.

The LD 63 for therapeutic light emission emits red laser light having a center wavelength of 665 nm. The red laser light is led into the optical fiber 64c through a not-shown lens and the like. The optical fiber 64c is integrated with the optical fiber 64b into the optical fiber 64d, and is then connected to the optical fiber 56B of the electronic endoscope 12 via the connector 18. Thus, the red laser light emitted from the LD 63 is led into the light projecting unit 55B. The red laser light is diffused by the light diffusing member 59, and is applied to the internal body part as the therapeutic light for the PDT.

The CPU 66 controls turn-on/off timings of each LD 61, 62, or 63. For example, the CPU 66 selectively turns on one of the LDs 62 and 63 based on the signal inputted from the CPU 41. Accordingly, one of the excitation light for the PDD and the therapeutic light for the PDT is applied from the light projecting unit 55B to the internal body part.

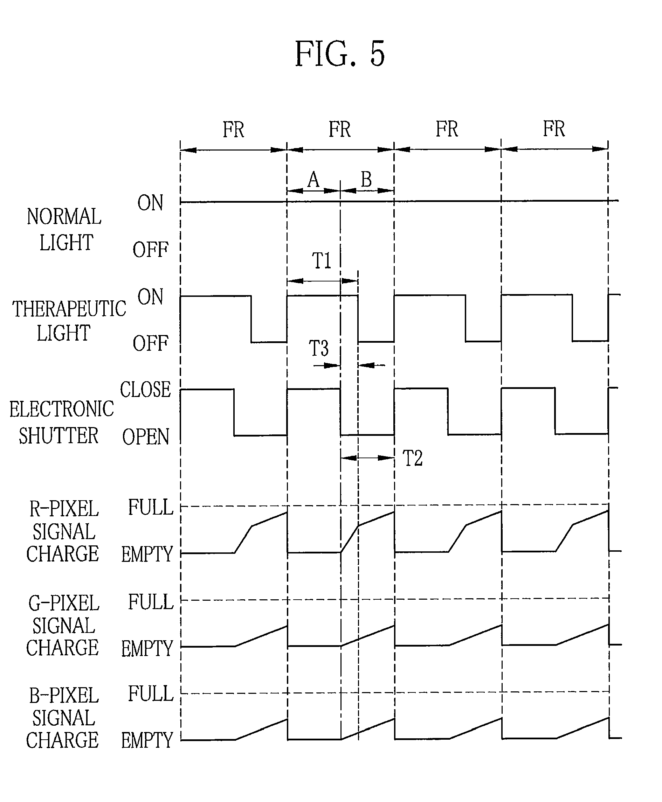

The CPU 66 applies the normal light, the excitation light, or the therapeutic light to the internal body part in accordance with the operation mode and in synchronization with drive timing of the CCD 21, by control of the turn-on/off timings of each LD 61, 62, or 63 in response to the regulation signal and the synchronization signal from the CPU 41. In the case of imaging the internal body part while performing the PDT, for example, the CPU 66 always turns on the LD 61 to keep emitting the normal light from the light projecting unit 55A, and concurrently turns on the LD 63 in a part of the single frame to emit the therapeutic light from the light projecting unit 55B. At this time, as shown in Fig. 5, the CPU 66 turns on the LD 63 in synchronization with a start point of the frame. After the LD 63 keeps emitting the therapeutic light for a predetermined time, the CPU 66 turns off the LD 63. The CPU 66 keeps the LD 63 turned on until it partly overlaps with the charge accumulation period corresponding to the second half of the frame. Information about the duration of the overlap (hereinafter called overlap period) between a therapeutic light irradiation period and the charge accumulation period is included in the regulation signal. The overlap period is determined by the CPU 41 based on settings and the ALC data so as to prevent occurrence of halation in the endoscopic image.

Furthermore, the CPU 66 communicates with the CPU 41 of the processor device 13, and controls emission amounts of the LDs 61 and 62 to regulate the amounts of the normal light and the excitation light to be applied to the internal body part. The CPU 66 automatically controls the light amount by the ALC in accordance with the luminance of the captured endoscopic image and the like based on the ALC data produced by the DSP 42.

A hood 81 is attached to the distal end portion 20 when performing the PDD and the PDT. The hood 81 is provided with an imaging window opening 82, a lighting window opening 83, and a medical instrument outlet opening in positions corresponding to the imaging window 23, the lighting window 24, and the medical instrument outlet provided in the distal end surface, respectively.

Into the imaging window opening 82, a light reducing filter 85 is fitted. The light reducing filter 85 is a filter (so-called notch filter) that reduces the excitation light and the therapeutic light, and transmits light at substantially 100% in a wavelength band other than those of the excitation light and the therapeutic light. As a result, in the PDD, the fluorescent light emitted from the photosensitive drug and the reflected excitation light are incident upon the imaging window 23, and only the reflected excitation light is reduced. The light reducing filter 85 facilitates obtainment of a clear image of the internal body part with the fluorescent light, even if the intensity of the fluorescent light is low.

In the PDT, on the other hand, the therapeutic light is applied at the highest possible power to minimize the treatment time. The reflected therapeutic light is reduced by the light reducing filter 85. Thus, the amount of the reflected therapeutic light reaching the CCD 21 is reduced.

Note that, the normal light is in a broad wavelength band, and contains a light component in a wavelength band of the excitation light and a light component in a wavelength band of the therapeutic light. Thus, if imaging (hereinafter called normal light imaging) is carried out under the normal light with the electronic endoscope 12 having the hood 81, the light reducing filter 85 cuts off the light components in the same wavelength bands as those of the excitation and therapeutic light, but transmits major light components in the other wavelength bands. Therefore, the endoscopic image (hereinafter called normal light image) captured under the normal light hardly changes irrespective of the presence or absence of the hood 81.

Into the lighting window opening 83, a transparent member (for example, glass plate) that transmits at least the normal light and the excitation light is fitted. Thus, if the hood 81 is attached to the distal end portion 20, the normal light and the excitation light are applied to the internal body part as in the case of no hood 81.

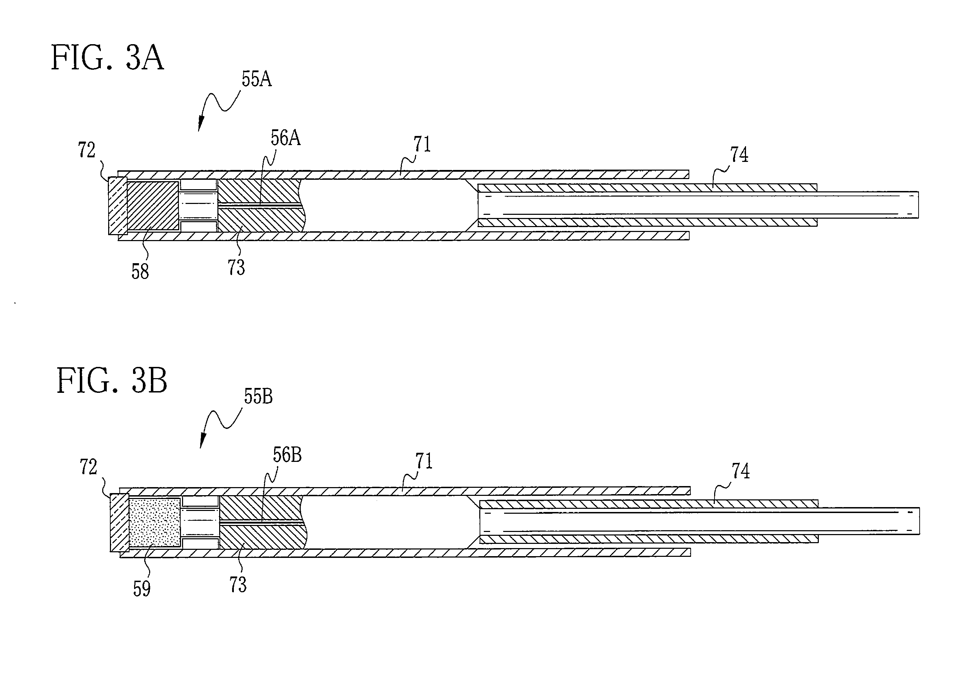

As shown in Fig. 3A, the light projecting unit 55A is provided with the phosphor 58, a cylindrical fixed sleeve 71 for covering the periphery of the phosphor 58, a protective glass plate 72 for sealing a front end of the fixed sleeve 71, and a ferrule 73 for holding the optical fiber 56A inserted into the fixed sleeve 71 at a central axis of the fixed sleeve 71. The optical fiber 56A extends from a rear end of the ferrule 73, and passes through the flexible sleeve 74. The light projecting unit 55B, as shown in Fig. 3B, has the same configuration as that of the light projecting unit 55A, except to have the light diffusing member 59 instead of the phosphor 58, and have the optical fiber 56B instead of the optical fiber 56A.

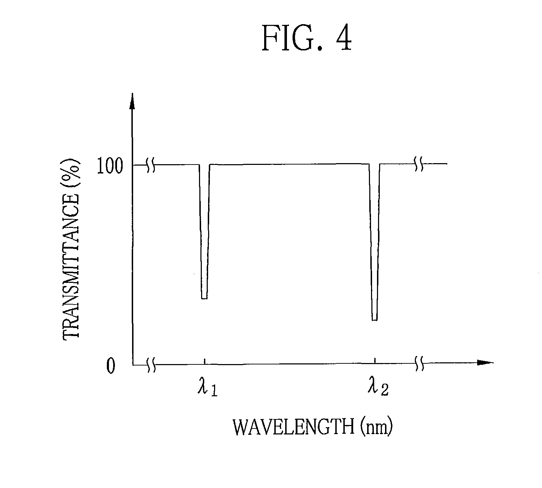

As shown in Fig. 4, the light reducing filter 85 has low transmittance at a wavelength near λ₁ of the excitation light and at a wavelength near λ₂ of the therapeutic light, while it transmits light having the other wavelengths at substantially 100%. The light reducing filter 85 is constituted of a first notch filter having low transmittance at the wavelength near λ₁ of the excitation light and a second notch filter having low transmittance at the wavelength near λ₂ of the therapeutic light, which are overlaid one another. Thus, the transmittance at the wavelength λ₁ of the excitation light is determined depending on the amount of the excitation light used in the PDD and the amount of the produced fluorescent light. The transmittance at the wavelength λ₂ of the therapeutic light is determined depending on the amount of the therapeutic light used in the PDT. Note that, the light reducing filter 85 is not necessarily composed of a combination of the two notch filters, but may be composed of one or more optical films having arbitrary concrete configuration and the like, as long as it can reduce the amounts of the excitation light and the therapeutic light.

The operation of the electronic endoscope system 11 having the above configuration will be described. In the case of performing the PDD and the PDT with the electronic endoscope system 11, the doctor administers the photosensitive drug in advance to the patient by an intravenous injection or the like. After a lapse of predetermined time from the administration, when the photosensitive drug is sufficiently accumulated in tumor cells, examination and treatment are started using the electronic endoscope system 11. As a preparation to the examination and treatment, the doctor connects the electronic endoscope system 12 to the processor device 13 and the light source device 14, and turns on the processor device 13 and the light source device 14. Also, the doctor inputs the information related to the patient from the operation unit 51. The hood 81 is attached to the distal end portion 20 of the electronic endoscope 12. Then, the insert section 16 is introduced into the patient's body cavity, while the inside of the patient's body is imaged under the normal light.

When the distal end portion 20 reaches the body part to be examined, the electronic endoscope system 11 is switched from the normal imaging mode to the PDD mode by operation on the operation section 17. In response to switching to the PDD mode, the CPU 41 inputs the regulation signal and the synchronization signal for the PDD to the CPU 29 of the electronic endoscope 12 and the CPU 66 of the light source device 14. Upon the input of the regulation signal and the synchronization signal, the CPU 29 of the electronic endoscope 12 drives the CCD 21 to image the internal body part, just as in the case of the normal light imaging. Concurrently, the CPU 66 of the light source device 14 turns off the LD 61 for normal light emission, and turns on the LD 62 for excitation light emission. Therefore, the excitation light is applied from the light projecting unit 55B to the internal body part, instead of the normal light from the light projecting unit 55A.

By application of the excitation light, the photosensitive drug accumulated in the tumor cells emits the fluorescent light. Thus, a PDD image that puts emphasis on the tumor cells with the fluorescent light is captured and displayed on the monitor 22. The doctor examines the position, size, shape and the like of the tumor cells by alternately or concurrently watching the normal light image and the PDD image. After that, the position and direction of the distal end portion 20 are adjusted so that the therapeutic light is efficiently applied to the tumor cells.

After the adjustment of the position and direction of the distal end portion 20, the doctor steps on a pedal switch (operation unit 51) to start the PDT. Upon a press of the pedal switch, the CPU 41 inputs the regulation signal and the synchronization signal for the PDT to the CPU 29 of the electronic endoscope 12 and the CPU 66 of the light source device 14. In response to the regulation signal and the synchronization signal for the PDT, the CPU 29 of the electronic endoscope 12 adjusts the open/close timings of the electronic shutter of the CCD 21, and the CPU 66 of the light source device 14 adjusts the turn-on/off timings of each LD 61, 62, or 63, to switch the operation mode to the PDT mode.

As shown in Fig. 5, the CPU 29 of the electronic endoscope 12 commands the TG 26 to input the predetermined clock signal to the CCD 21, so that the CCD 21 closes the electronic shutter in a first half A of a single frame FR, and opens the electronic shutter in a second half B of the frame FR. Thereby, the CCD 21 accumulates the signal charges during the second half B of the frame FR.

In response to the input of the regulation signal and the synchronization signal for the PDT, the CPU 66 of the light source device 14 turns on the LD 61 to start continuous emission of the normal light, and turns off the LD 62 for excitation light emission. Accordingly, the normal light is continuously emitted from the light projecting unit 55A during the performance of the PDT. At the same time, the CPU 66 cyclically turns on and off the LD 63 for the sake of obtaining pulse emission of the therapeutic light from the light projecting unit 55B in synchronization with the drive timing of the CCD 21. The LD 63 is turned on concurrently with a start of the frame FR at which the electronic shutter of the CCD 21 is closed, and is turned off in the middle of the second half B of the frame FR.

As a result, a therapeutic light irradiation period T1 and a charge accumulation period T2 has an overlap period T3. The duration of the overlap period T3 is predetermined by the CPU 41 in accordance with the settings and the like, so that the halation does not occur in an image (hereinafter called PDT image) captured during the PDT. The therapeutic light irradiation period T1 is set equal to the sum of the overlap period T3 and the first half A of the frame FR.

In each pixel of the CCD 21, a signal charge accumulation amount is zero (empty) in the first half A of the frame FR in which the electronic shutter is closed. After that, when the electronic shutter is opened, the signal charges are accumulated in accordance with the amount of incident light. At this time, in each green pixel (hereinafter called G-pixel), signal charges are accumulated in response to a green component of the reflected normal light by an amount proportionate to the duration of the charge accumulation period T2. Since the therapeutic light is red, the therapeutic light does not have an influence on the charge accumulation amount of the G-pixel even in the overlap period T3, and only the reflected normal light is converted into the signal charges in the G-pixel. Likewise, in each blue pixel (B-pixel), signal charges are accumulated in response to a blue component of the reflected normal light by an amount proportionate to the duration of the charge accumulation period T2.

In each red pixel (R-pixel), on the other hand, when the electronic shutter is opened, signal charges are accumulated in response to both a red component of reflected therapeutic light and a red component of the reflected normal light during the overlap period T3, being a beginning of the charge accumulation period T2. For this reason, the signal charge accumulation amount of the R-pixel increases more sharply than those of the G- and B-pixels, due to the entry of the reflected therapeutic light. For the rest of the charge accumulation period T2 after the overlap period T3, the signal charge accumulation amount increases in proportion to the red component of the reflected normal light. The duration of the overlap period T3 is determined in such a range that the signal charge accumulation amount of the R-pixel does not become full (a maximum permissible accumulation amount) at time of completing the charge accumulation period T2. Also, in R-pixels situated in a position where the reflected therapeutic light does not enter, signal charges are accumulated in response to only the red component of the reflected normal light, as in the case of the G- and B-pixels. Thus, the signal charge accumulation amount of those R-pixels is in proportion to the amount of the reflected normal light throughout the charge accumulation period T2.

As described above, in the PDT mode, the first half A of the frame FR and a part of the second half B continued from the first half A are assigned to the therapeutic light irradiation period T1, and the second half B of the frame FR is assigned to the charge accumulation period T2. More specifically, the therapeutic light irradiation period T1 and the charge accumulation period T2 partly overlap at the overlap period T3. The duration of the overlap period T3 is determined in such a range that the halation does not occur in the PDT image. Therefore, with the use of the electronic endoscope system 11, it is possible to favorably watch the internal body part in the endoscopic image even during the performance of the PDT.

When compared with a comparative example 1 where a therapeutic light irradiation period and a normal light irradiation period are established alternately without having an overlap period (T1=T2 and T3=0), the present invention is superior to the comparative example 1 because the tumor cells are irradiated with the therapeutic light for longer time by the duration of the overlap period T3 in each frame FR. The PDT often takes long treatment time of one hour or more, even if the therapeutic light is continuously applied irrespective of the drive cycle of the CCD 21. Against this backdrop, the electronic endoscope system 11 according to the present invention can shorten the treatment time by several tens of minutes, though it depends on the duration of the overlap period T3 and the total amount of the therapeutic light necessary for the treatment. This results in reducing the burden of the patient.

According to the electronic endoscope system 11, due to the establishment of the overlap period T3, the PDT image captures an image in which an irradiation spot of the therapeutic light is overlaid on the normal light image. The intensity of the irradiation spot is proportional to the duration of the overlap period T3. In the case of the comparative example 1, since the therapeutic light irradiation period and the normal light irradiation period are established alternately without having the overlap period, it is difficult to judge from the normal light image displayed on the monitor 22 whether or not the therapeutic light is properly emitted and whether or not the tumor cells are appropriately irradiated with the therapeutic light. However, according to the electronic endoscope system 11 of the present invention, it is possible to favorably watch the internal body part to be treated during the performance of the PDT, and additionally, to grasp the irradiation spot of the therapeutic light in the PDT image.

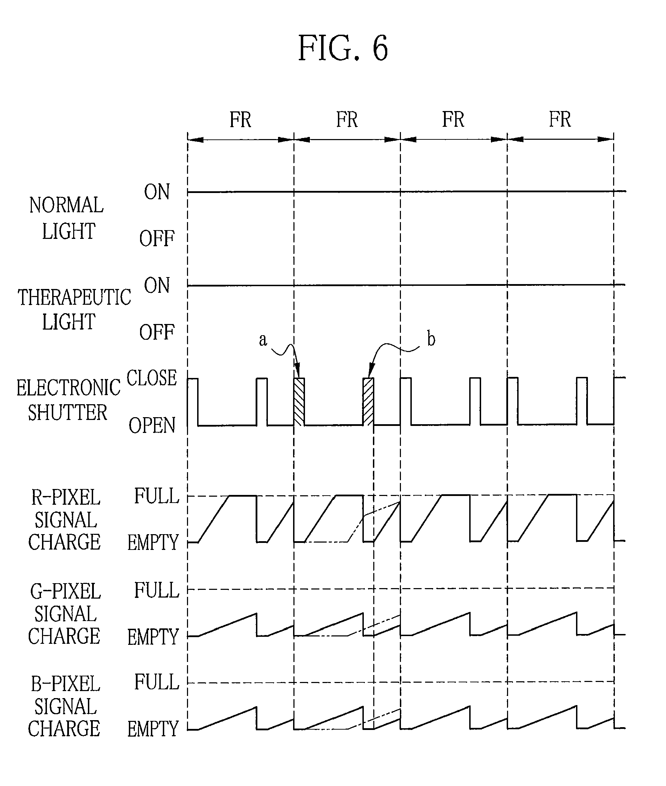

Furthermore, the electronic endoscope system 11 according to the present invention has the following merits, in comparison with a comparative example 2. Note that, in the comparative example 2, as shown in Fig. 6, the therapeutic light is continuously applied irrespective of a drive cycle of the CCD 21, and the electronic shutter is closed in the middle of the frame FR to abandon accumulated signal charges and prevent occurrence of the halation in an image captured during the PDT.

In the comparative example 2, the electronic shutter is closed at a start point "a" of the frame FR, and opened at once to start accumulation of the signal charges. After that, the electronic shutter is closed again in the middle "b" of the frame FR to abandon the signal charges from every pixel. Between the start point "a" and the middle point "b", the R-pixels become full by accumulation of the reflected therapeutic light. However, since the signal charges are abandoned from every pixel in the middle "b" of the frame FR, it is possible to prevent occurrence of the halation due to the therapeutic light.

Here, the timing of the point "b" of closing the electronic shutter is determined such that the amount of the signal charges accumulated in each R-pixel after the point "b" in the comparative example 2 becomes equal to the amount of the signal charges accumulated in the R-pixel in the electronic endoscope system 11 of the present invention, as illustrated by a chain double-dashed line in Fig. 6. In this case, according to the comparative example 2, the electric shutter is closed at the point "b", and the signal charges of the G- and B-pixels are abandoned just as with those of the R-pixels. Thus, the amount of the signal charges accumulated in the G- and B-pixels of the comparative example 2 is less than that of the present invention, as shown by chain double-dashed lines in rows of the G- and B-pixels in Fig. 6. Also, the R-pixels without receiving the reflected therapeutic light accumulate a little amount of the signal charges, as in the case of the G- and B-pixels. Accordingly, in the PDT image taken in the comparative example 2, only the red irradiation spot of the therapeutic light seems to emerge. Thus, it is difficult to favorably watch the entire part to be treated during the performance of the PDT. In contrast to the comparative example 2, the electronic endoscope system 11 according to the present invention can favorably image the entire part in full color. Although the electronic endoscope system 11 requires longer treatment time in the PDT than that of the comparative example 2 due to extinguishment of the therapeutic light, it is possible to favorably watch the internal body part with a minimum extension of the treatment time.

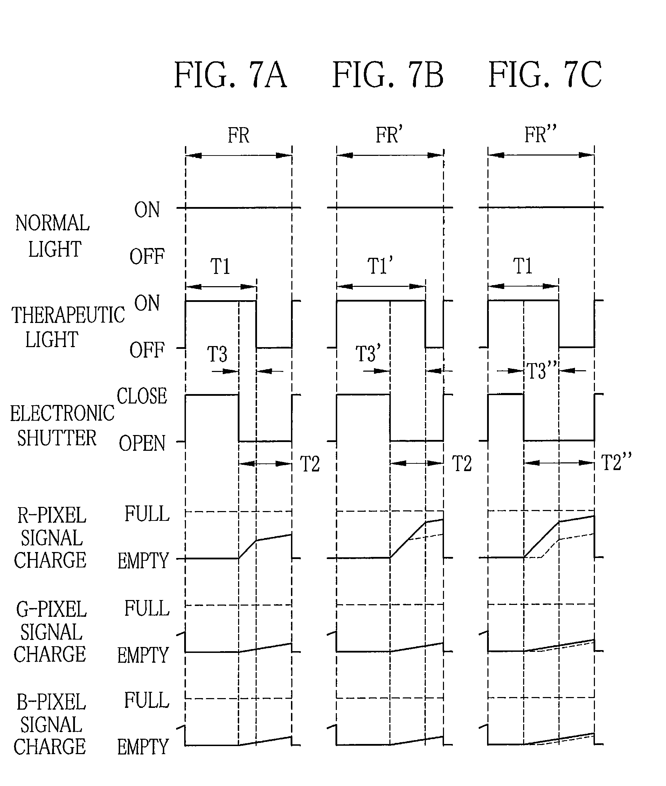

In the above first embodiment, the overlap period T3 is established in advance by the settings and the like, but the duration of the overlap period T3 may be variable. For example, if the amount of the signal charges accumulated in the R-pixels is small and the PDT image is dark with setting the overlap period at T3 as shown in Fig. 7A, the duration of the overlap period may be extended to T3' by extension of the therapeutic light irradiation period to T1' in each frame as shown in Fig. 7B, or the duration of the overlap period may be extended to T3" by extension of the charge accumulation period to T2" as shown in Fig. 7C. On the contrary, when the PDT image is too bright, the overlap period T3 may be shortened in a like manner.

The extension of the overlap period T3 is carried out as follows, based on the ALC data inputted from the DSP 42 to the CPU 41. Note that, description of shortening the overlap period T3 will be omitted because it is similar to the description of the extension. The ALC data is inputted from the DSP 42 to the CPU 41. The charge accumulation period T2 is set shorter in the PDT mode than that in the normal imaging mode, so the PDT image is often judged to be dark as compared to the normal light image. For this reason, during the PDT, the ALC data mostly directs to increase the amount of the normal light and take a brighter image. Here, in response to this direction, the CPU 66 of the light source device 14 increases the emission amount of the normal light to its maximum.

Upon the input of the ALC data, the CPU 41 converts an increase in the amount of the normal light directed by the ALC data into the duration of the overlap period T3 according to a predetermined expression (or data table or the like), and determines a new overlap period T3' or T3". Then, a new regulation signal and a new synchronization signal are inputted to the CPU 29 of the electronic endoscope 12 and the CPU 66 of the light source device 14, so that the therapeutic light irradiation period T1 is extended to T1' (T1'>T1) or the charge accumulation period T2 is extended to T2' (T2'>T2) in accordance with the newly determined overlap period T3' or T3". The CPU 29 of the electronic endoscope 12 and the CPU 66 of the light source device 14 control the CCD 21 and each LD 61, 62, or 63 based on the newly inputted regulation signal and synchronization signal. In this manner, the overlap period T3 is extended to T3' or T3" in the next and later frames.

When the overlap period is extended from T3 to T3' by extending the therapeutic light irradiation period from T1 to T1' in a frame FR' as shown in Fig. 7B, the amount of the therapeutic light applied per frame is increased by the extension of the overlap period, and the amount of the signal charges accumulated in the R-pixels is increased. On the other hand, since the charge accumulation period T2 is not changed from that of the prior frame FR, the amount of the signal charge accumulated in the G- and B-pixels is substantially the same as that of the prior frame FR. The same goes for the R-pixels without receiving the reflected therapeutic light. Thus, it is possible to shorten the treatment time, and take the PDT image having the brighter red irradiation spot of the therapeutic light. This method is effective at further reducing the treatment time, in such a case where the patient is short on physical strength.

In the case of extending the overlap period from T3 to T3" by extension of the charge accumulation period from T2 to T2" in a frame FR" as shown in Fig. 7C, the amount of signal charges accumulated in the R-pixels is increased due to the extension of the overlap period. Likewise, the amount of signal charges accumulated in the G- and B-pixels is increased too. Thus, it is possible to take the brighter PDT image in all of RGB colors. This is particularly useful for watching the detailed properties of the body part including a treatment effect and the like, concurrently with the performance of the PDT.

In this embodiment, one of the therapeutic light irradiation period T1 and the charge accumulation period T2 is varied to extend or shorten the overlap period T3. Instead, both the therapeutic light irradiation period T1 and the charge accumulation period T2 may be varied to extend or shorten the overlap period T3. In this case, the CPU 41 may initially determine a target duration of the overlap period T3 based on the ALC data, and then determine the duration of the therapeutic light irradiation period T1 and the charge accumulation period T2 so as to minimize an increase or decrease of the determined target duration of the overlap period T3, the therapeutic light irradiation period T1, and the charge accumulation period T2. When the overlap period T3 is adjusted by varying both the therapeutic light irradiation period T1 and the charge accumulation period T2, the treatment time and brightness of the PDT image are optimized.

In the above first embodiment, the therapeutic light irradiation period T1 is established in the first half of the frame FR, and the charge accumulation period T2 is established in the second half thereof, and the overlap period T3 is established across a front end of the charge accumulation period T2 and a last end of the therapeutic light irradiation period T1. However, the charge accumulation period T2 may be established in the first half of the frame FR, and the therapeutic light irradiation period T1 may be established in the second half thereof. In this case, the overlap period T3 may be established across a front end of the therapeutic light irradiation period T1 and a last end of the charge accumulation period T2.

### (Second Embodiment)

In the above first embodiment, the PDT image is taken under the normal light during the performance of the PDT, but an image (hereinafter called PDD image) of the fluorescent light of the PDD may be taken instead during the PDT. In this case, if both the PDT image and the PDD image are taken in each frame, the treatment time is doubled. To reduce the treatment time, the following second embodiment is preferably performed.

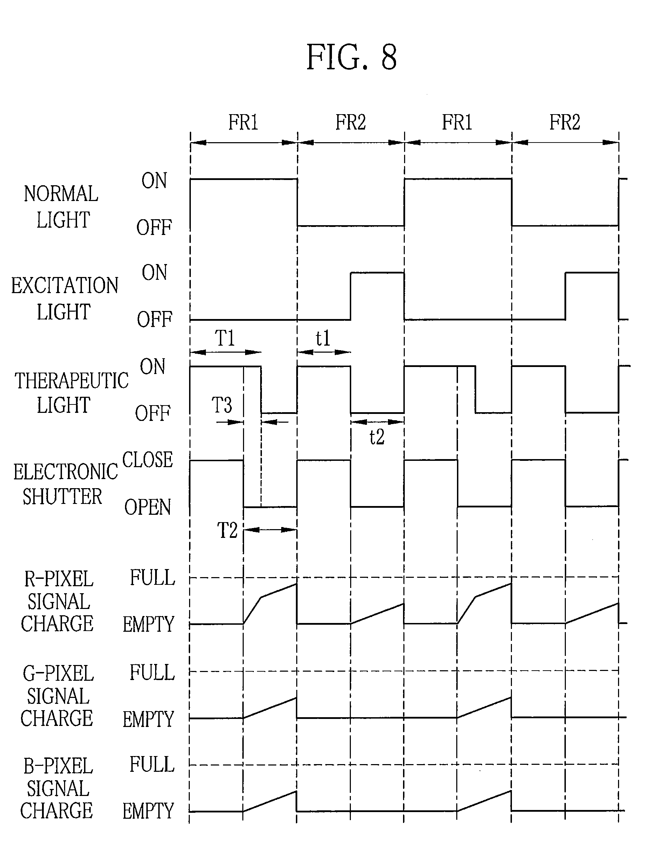

The fluorescent light emitted from the photosensitive drug upon application of the excitation light for the PDD is red light having a center wavelength of 660 nm, for example, while the therapeutic light is red light having a center wavelength of 665 nm. The amount of the fluorescent light is extremely small as compared with that of the therapeutic light. For these reasons, if any slight amount of therapeutic light is applied in capturing the PDD image, the fluorescent light is buried in the reflected therapeutic light, and the tumor cells cannot be distinguished in the taken PDD image. Thus, as shown in Fig. 8, a frame FR1 for taking the PDT image and a frame FR2 for taking the PDD image are carried out alternately.

In the frame FR1 for taking the PDT image, just as with the above first embodiment, the overlap period T3 is provided across the therapeutic light irradiation period T1 and the charge accumulation period T2. This makes it possible to take the favorable PDT image in which the internal body part to be treated can be watched clearly under irradiation with the normal light, while the treatment time is minimized.

In a frame FR 2 for taking the PDD image, the CPU 29 of the electronic endoscope 12 closes the electronic shutter in a first half t1 of the frame FR2, and opens the electronic shutter in a second half t2 of the frame FR2. Note that, the duration of the first half t1 is equal to that of the second half t2 (i.e. t1=t2) . The CPU 66 of the light source device 14 turns off the LD 61 to stop emission of the normal light from the light projecting unit 55A throughout the whole duration (t1+t2) of the frame FR2. The CPU 66 of the light source device 14, on the other hand, turns on the LD 63 to apply the therapeutic light to the internal body part through the light projecting unit 55B in the first half t1 of the frame FR2. In the second half t2 of the frame FR2, the CPU 66 turns off the LD 63, and turns on the LD 62 instead to apply the excitation light to the internal body part through the light projecting unit 55B. In such a manner, in the frame FR2 for taking the PDD image, the PDT treatment is performed in the first half t1, and the PDD image is captured in the second half t2. The PDD image taken in the frame FR2 is displayed on the monitor 22 together with the PDT image taken in the frame FR 1 in a tiled manner. The display on the monitor 22 may be switched between the PDD image and the PDT image in response to operation on the operation unit 51.

As described above, it is possible to prevent extension of the treatment time of the PDT, while the PDT image and the PDD image are taken approximately at the same time.

In the second embodiment, the therapeutic light is applied in the first half of the frame FR2, and the PDD image is taken in the second half thereof. However, just as with a modification example of the first embodiment in which the overlap period T3 is varied, the therapeutic light irradiation period and the charge accumulation period are preferably varied in the frame FR2 based on the ALC data and the like obtained from the taken PDD image. If the therapeutic light irradiation period and charge accumulation period are variable in the frame FR2, both the short treatment time and the favorable PDD image can be easily obtained at the same time.

In the second embodiment, the PDT image is taken in the frame FR1, as in the case of the first embodiment. However, for example, the therapeutic light may be applied throughout the whole frame FR1 and the first half t1 of the frame FR2, and the PDD image may be taken in the second half t2 of the frame FR2. In this case, although the halation occurs in the PDT image taken in the frame FR1, the treatment time is further reduced. This method is suitable in a case where the body part to be treated and the tumor cells are clearly seen in the PDD image, and the further reduction of the treatment time is desired.

In the second embodiment, the frame FR1 for taking the PDT image and the frame FR2 for taking the PDD image are alternately carried out, but the frequency of taking the PDD image is arbitrary changeable. For example, the PDT images may be taken over several times in succession, and thereafter the single PDD image may be taken.

Note that, in the above second embodiment, the therapeutic light is applied in the first half t1 of the frame FR2, and the second half t2 is assigned to the charge accumulation period. However, the first half t1 of the frame FR2 may be assigned to the charge accumulation period to take the PDD image, and the therapeutic light may be applied in the second half t2 of the frame FR2.

In the above first and second embodiments, the LDs are used as the light sources, but another type of well-known light source, such as an LED or a halogen lamp, may be used instead.

In the above first and second embodiments, the three types of light, that is, the normal light, the excitation light for the PDD, and the therapeutic light for the PDT are applied to the internal body part, but another type of light may be used in combination. For example, use of blue light having a center wavelength of 472 nm allows extraction of information about an oxygen saturation level in blood and the depth of a blood vessel. Also, use of infrared light having a center wavelength of 785 nm allows observation of IGC (indocyanine green) injected into a blood vessel. Use of ultraviolet light having a center wavelength of 375 nm allows fluorescence observation employing luciferase. If the excitation light for the PDD is used as illumination light for lighting the internal body part without administration of the photosensitive drug, so-called narrow-band imaging may be carried out.

In the above first and second embodiments, the light source device 14 is provided with the three types of LDs 61 to 63. It is preferable that each light source is composed of plural LDs, and light from the plural LDs are combined by a coupler or the like for use in lighting. If each light source is composed of the plural LDs, as described above, it is possible to reduce variations in an emission wavelength among the LDs and prevent occurrence of speckle.

Note that, in the above first and second embodiments, the electronic endoscope 12 is provided with the single light projecting unit 55A for emitting the normal light and the single light projecting unit 55B for emitting the excitation light or the therapeutic light, but may be provided with three or more light projecting units in total having the same configuration. In the case of using light other than the normal light, the excitation light for the PDD, and the therapeutic light for the PDT as illumination light or the like, a specific light projecting unit may be additionally provided. Note that, however, it is preferable to share the smallest possible number of light projecting units for the sake of reducing the diameter of the insert section 16.

In the above first and second embodiments, each of the LDs 61 to 63 is continuously turned on or off. However, when applying the normal light, the excitation light, or the therapeutic light to the internal body part, each LD 61, 62, or 63 may be turned on and off instantaneously, and the internal body part may be pulse-irradiated with the desired light in the same light amount as that in the case of the continuous irradiation.

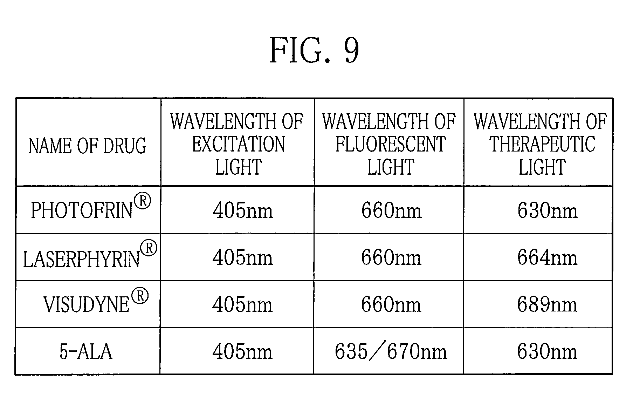

Note that, an arbitrary drug is usable as the photosensitive drug for the PDD and PDT, as long as the drug accumulates in a portion (tumor cells or the like) to be treated, and emits fluorescent light upon application of excitation light, and is effective at treating the portion by application of the therapeutic light. The wavelengths of the excitation light and the therapeutic light are determined in response to the type of photosensitive drug to be used. In the case of using the plural types of photosensitive drugs that require the excitation light and the therapeutic light with different wavelengths, plural light sources specific to the individual drugs may be provided. Fig. 9 shows a table of examples of the wavelengths of the excitation light for the PDD, the fluorescent light emitted upon application of the excitation light, and the therapeutic light for the PDT, which differ from drug to drug. Even if any of photofrin®, laserphyrin®, visudyne®, and 5-ALA (aminolevulinic acid) is used as the photosensitive drug, laser light having center wavelengths of 350 to 450 nm is available as the excitation light for the PDD, and specifically, laser light having a center wavelength of 405 nm is preferably available. The 5-ALA is converted into protoporphyrin IX, which is accumulated in the tumor cells. A wavelength ratio of the fluorescent light emitted from the protoporphyrin IX varies in accordance with the stage of disease.

In the above first and second embodiments, an image taken in the single frame is the so-called progressive image, which is produced from the signal charges of all pixels. However, the PDT image and the PDD image may be taken by interlaced scanning, in which each image is produced from the signal charges of pixels in every other line of the CCD 21 on a field basis.

In the above first and second embodiments, the CCD 21 is used as an image sensor, but another type of well-known image sensor such as a CMOS image sensor is suitably usable instead. In the above first and second embodiments, each pixel of the CCD 21 is provided with the R, G, or B color filter, and the CCD 21 captures a full-color image by a so-called simultaneous method. However, an image may be captured by a frame sequential method, in which a filter having R, G, and B color segments is rotated on a surface of the image sensor, and each color image is obtained sequentially. In adopting the frame sequential method, the durations of the therapeutic light irradiation period T1, the charge accumulation period T2, and the overlap period T3 can be determined independently from color to color. For example, when taking a red color image, the charge accumulation period T2 and the overlap period T3 are set short to prevent occurrence of the halation. When taking green and blue color images, on the other hand, the charge accumulation period T2 is appropriately adjustable irrespective of the duration of the therapeutic light irradiation period T1, because the filter cuts off the therapeutic light. In the case of adopting the frame sequential method, since the therapeutic light irradiation period T1, the charge accumulation period T2, and the overlap period T3 are determined independently, it is possible to further easily obtain the favorable normal light image (PDT image) during the performance of the PDT.

In the above first and second embodiments, the light source device 14 has the LD 63 for therapeutic light emission, and the therapeutic light is emitted from the light projecting unit 55B provided in the insert section 16, but a way to apply the therapeutic light to the internal body part is not limited to it. For example, a therapeutic light source may be provided separately from the light source device 14. A light guiding probe is inserted from the medical instrument insertion port into the channel such that a distal end of the light guiding probe protrudes from the medical instrument outlet. The therapeutic light may be led through the light guiding probe and applied to the internal body part from the distal end of the light guiding probe.

In the above first and second embodiments, the normal light remains turned on during the therapeutic light irradiation period T1. In the frame for taking the PDT image, the normal light has to be applied in the charge accumulation period T2 (including the overlap period T3), but is unnecessary in the therapeutic light irradiation period T1 except the overlap period T3. The normal light may be turned off in that period.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. An electronic endoscope system (11) comprising:
a normal light emitting means (61) for emitting white light to an internal body part to be imaged;
a therapeutic light emitting means (63) for emitting therapeutic light to said body part to treat a lesion emerging in said body part;
an imaging means (21) for forming an image of said body part, said imaging means having a plurality of pixels each of which makes photoelectric conversion of incident light and accumulates signal charges of an amount corresponding to an amount of said incident light; and
a control means (29, 66, 41) for controlling said normal light emitting means (61), said therapeutic light emitting means (63), and said imaging means (21), such that a therapeutic light irradiation period (T1) in which said body part is irradiated with said therapeutic light and a first charge accumulation period (T2) in which said signal charges are accumulated under irradiation of said white light overlap each other.

2. The electronic endoscope system (11) according to claim 1,
wherein said control means (29, 66, 41) establishes said therapeutic light irradiation period (T1) and said first charge accumulation period (T2) in an alternate manner; and
wherein an overlap period (T3) is set up between said therapeutic light irradiation period (T1) and said first charge accumulation period (T2) at timing of transition from said therapeutic light irradiation period (T1) to said first charge accumulation period (T2), or at timing of transition from said first charge accumulation period (T2) to said therapeutic light irradiation period (T1).

3. The electronic endoscope system (11) according to claim 2, wherein a duration of said overlap period (T3) is variable, and determined by said control means (29, 66, 41).

4. The electronic endoscope system (11) according to claim 3,
wherein said control means (29, 66, 41) includes a brightness judging means (41) for judging brightness of said image captured by said imaging means (21); and
wherein said control means (29, 66, 41) determines said duration of said overlap period (T3) based on a judgment result of said brightness judging means (41).

5. The electronic endoscope system (11) according to claim 4, wherein said control means (29, 66, 41) adjusts a duration of said therapeutic light irradiation period (T1) to vary said duration of said overlap period (T3).

6. The electronic endoscope system (11) according to claim 1, further comprising:
a special light emitting means (62) for applying special light in a narrow wavelength band to said internal body part; and
wherein said control means (29, 66, 41) controls said special light emitting means (62) and said imaging means (21), so as to establish a second charge accumulation period (t2) in which said signal charges are accumulated under irradiation of said special light.

7. The electronic endoscope system (11) according to claim 6,
wherein said special light is excitation light for causing a photosensitive drug accumulated in said body part to emit fluorescent light; and
wherein said imaging means (21) captures an image of said fluorescent light emitted from said photosensitive drug in said body part during said second charge accumulation period (t2).

8. The electronic endoscope system (11) according to claim 7, wherein said control means (29, 66, 41) controls said special light emitting means (62) , said therapeutic light emitting means (63), and said imaging means (21), such that said therapeutic light irradiation period (t1) and said second charge accumulation period (t2) do not overlap each other.

9. The electronic endoscope system (11) according to claim 8, wherein while said lesion is treated by application of said therapeutic light, both imaging under irradiation with said white light and imaging under emission of said fluorescent light are carried out.

10. The electronic endoscope system (11) according to claim 9, wherein said imaging under irradiation with said white light and said imaging under emission of said fluorescent light are alternately carried out.

11. The electronic endoscope system (11) according to claim 8, wherein said control means (29, 66, 41) establishes both a first frame (FR1) and a second frame (FR2) in a treatment process of said lesion, and said first frame (FR1) includes said therapeutic light irradiation period (T1) and said first charge accumulation period (T2) with having said overlap period (T3) between said therapeutic light irradiation period (T1) and said first charge accumulation period (T2), and said second frame (FR2) includes said therapeutic light irradiation period (t1) and said second charge accumulation period (t2) without having an overlap period between said therapeutic light irradiation period (t1) and said second charge accumulation period (t2).

12. The electronic endoscope system (11) according to claim 11, wherein said first frame (FR1) and said second frame (FR2) are carried out in an alternate manner.
